(19) Europäisches Patentamt
European Patent Office
Office européen des brevets

(11) Veröffentlichungsnummer: **0 029 011**
**A1**

(12) **EUROPÄISCHE PATENTANMELDUNG**

(21) Anmeldenummer: 80810342.8

(22) Anmeldetag: 07.11.80

(51) Int. Cl.$^3$: **C 07 D 231/12**
A 01 N 43/56

(30) Priorität: 13.11.79 CH 10125/79

(43) Veröffentlichungstag der Anmeldung: 20.05.81 Patentblatt 81/20

(84) Benannte Vertragsstaaten: BE CH DE FR GB IT LI NL

(71) Anmelder: CIBA-GEIGY AG
Patentabteilung Postfach
CH-4002 Basel(CH)

(72) Erfinder: Moser, Hans, Dr.
Blumenrain 3
CH-4465 Magden(CH)

(54) **N-(Azolyl-1-äth-1'-yl)-halogenacetanilide, ihre Herstellung und ihre Verwendung als Herbizide.**

(57) N-Azolyl-1- äthyl-N-halogenacetanilide der Formel I

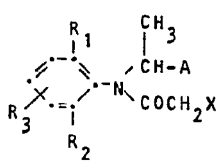

(I),

worin A einen über ein Stickstoffatom gebundenen, gegebenenfalls durch $C_1$-$C_4$ Alkyl, Cyano oder Halogen substituierten Azolring, X Chlor oder Brom und mindestens einer der Substituenten $R_1$, $R_2$ und $R_3$, $C_1$-$C_4$ Alkoxy, Halogen oder Halogenmethyl, die anderen auch Wasserstoff oder $C_1$-$C_4$ Alkyl bedeuten und deren Säureadditionssalze haben herbizide und das Pflanzenwachstum hemmende Eigenschaften. Sie eignen sich zum Einsatz als Selektivherbizide in Kulturpflanzungen.

EP 0 029 011 A1

BEZEICHNUNG GEÄNDERT
siehe Titelseite

CIBA-GEIGY AG
Basel (Schweiz)

5-12601/-

BEZEICHNUNG GEÄNDERT
siehe Titelseite

N-(Azolyl-1-äth-1'-yl)-N-halogenacetanilide

Die vorliegende Erfindung betrifft neue, herbizid wirksame und das Pflanzenwachstum regulierende N-(Azolyl-1-äth-1'-yl)-N-halogenacetanilide, Verfahren zu ihrer Herstellung, Mittel,welche diese N-Azolyl-1-äthyl-N-halogenacetanilide als Wirkstoffe enthalten, sowie deren Verwendung als Herbizide und zur Hemmung des Pflanzenwachstums.

Die erfindungsgemässen N-(Azolyl-1-äth-1'-yl)-N-halogenacetanilide entsprechen der Formel I

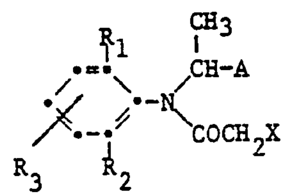

(I) ,

worin A einen über ein Stickstoffatom gebundenen Pyrazol-, Imidazol-, 1,2,4-Triazol- oder 1,3,4-Triazolring bedeutet, der durch $C_1$-$C_4$-Alkyl, Cyan oder Halogen substituiert sein kann, X ein Chlor- oder Bromatom und mindestens einer der Substituenten $R_1$, $R_2$, $R_3$, $C_1$-$C_4$-Alkoxy, Halogen oder Halogenmethyl, die andern auch noch Wasserstoff oder $C_1$-$C_4$ Alkyl bedeuten. Die Erfindung umfasst ebenfalls die Säureadditionssalze dieser N-(Azolyl-1-äth-1'-yl)-N-halogenacetanilide.

Bevorzugt wegen ihrer Wirkung sind die N-(Pyrazolyl-1-äth-1'-yl)-N-chloracetanilide der Formel Ia und ihre Säureadditionssalze

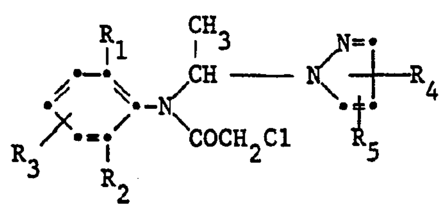

(Ia) ,

worin $R_1$, $R_2$ und $R_3$ die unter Formel I gegebene Bedeutung haben und $R_4$ und $R_5$ unabhängig voneinander je $C_1$-$C_4$-Alkyl, Cyan oder Halogen bedeuten.

Die erfindungsgemässen Acetanilide sind neue Verbindungen. Halogenacetanilide mit ähnlicher Struktur, d.h. solche, welche am Anilin-Stickstoff noch einen über eine Methylen-Brücke gebundenen Azol-Ring enthalten, sind kürzlich beschrieben worden, z.B. in den DOS No. 2 648 008, 2 704 281, 2 742 583 und 2 744 396.

Die neuen Verbindungen zeigen eine gute herbizide Wirkung, vor allem auf grasartige Unkräuter, wobei interessante Selektivitäten festgestellt wurden bei monokotylen Kulturen, vor allem bei Mais, aber auch bei einzelnen dikotylen Kulturpflanzen. Die neuen Verbindungen können sowohl im Vor- wie auch im Nachauflaufverfahren in mono- und dikotylen Kulturen als Unkrautmittel eingesetzt werden. Normalerweise werden Aufwandmengen, die zwischen 0,1 und 5 kg pro Hektar liegen, benötigt.

Ferner besitzen die Verbindungen der Formel I günstige wachstumsregulierende Effekte (Wuchshemmung). Insbesondere hemmen sie das Wachstum der Pflanzen. Beispiele für die nutzbringende Anwendung der erfindungsgemässen Verbindungen sind z.B:

- die Reduktion des vegetativen Wachstums bei Soja und andern Leguminosen, was zu einer Ertragssteigerung dieser Kultur führt;
- die Hemmung des unerwünschten Wachstums von Geiztrieben bei Tabak, dessen Haupttrieb man geschnitten hat, was der Ausbildung grösserer und schönerer Blätter zugute kommt;
- die Hemmung des Wachstums von Gras und dikotylen Pflanzen, wie Obstbäume, Zierbäume, Gebüsche und Hecken, zwecks Einsparung an Schnittarbeit.

Die neuen Verbindungen der Formel I werden erfindungsgemäss hergestellt, indem man die Schiff'sche Base eines Anilins entsprechend

der Formel II

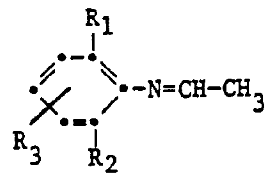

(II),

worin $R_1$, $R_2$ und $R_3$ die gegebene Bedeutung haben, in einem inerten aromatischen Lösungsmittel, zuerst mit der äquimolaren Menge eines Halogenacetylhalogenides der Formel III

$XCH_2COHal$ (III),

worin Hal und X unabhängig voneinander Chlor oder Brom bedeuten, und anschliessend mit einem N-Trimethylsilyl-azol der Formel IV

$(CH_3)_3Si-A$ (IV)

versetzt, worin A die oben gegebene Bedeutung hat, und gewünschtenfalls die erhaltene Verbindung in ein Säureadditionssalz umwandelt, respektive als solches aus der Reaktion isoliert.

Das Lösungsmittel soll gegenüber den Reaktionsteilnehmern inert sein. Es kommen beispielsweise in Frage: aliphatische oder aromatische Kohlenswasserstoffe, halogenierte Kohlenwasserstoffe, Aether oder Essigester.

Das sich bildende Trimethylchlorsilan wird durch Abdestillieren aus der Reaktionslösung entfernt. Das Zugeben des Halogenacetylhalogenides und des Trimethylsilylazols erfolgt vorteilhafterweise bei einer Temperatur von -60°C bis +30°C.

Die Schiff'sche Base der Formel II kann durch Zusammengeben eines Anilins der Formel V

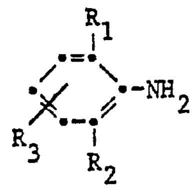

(V) ,

worin $R_1$, $R_2$ und $R_3$ die gegebene Bedeutung haben, mit Acetaldehyd in Benzol oder Toluol in Gegenwart eines wasserentziehendes Mittels erfolgen.

Die Herstellung der Trimethylsilyl-azole der Formel IV geschieht gemäss Birkhofer und Ritter, Ang. Chemie 77 (1965) S. 414 ff.

Die Wirkstoffe der Formel I sind stabile Verbindungen, welche in den üblichen organischen Lösungsmitteln, wie Alkoholen, Aethern, Ketonen, Dimethylsulfoxyd oder Dimethylsulfamid etc. löslich sind.

Die Herstellung erfindungsgemässer Mittel erfolgt in an sich bekannter Weise durch inniges Vermischen und Vermahlen von Wirkstoffen der allgemeinen Formel I mit geeigneten Trägerstoffen und/oder Verteilungsmitteln, gegebenenfalls unter Zusatz von gegenüber den Wirkstoffen inerten Antischaum-, Netz-, Dispersions- und/oder Lösungsmitteln. Die Wirkstoffe können in den folgenden Aufarbeitungsformen vorliegen und angewendet werden:

| | |
|---|---|
| Feste Aufarbeitungsformen: | Stäubemittel, Streumittel, Granulate, Umhüllungsgranulate, Imprägniergranulate und Homogengranulate; |
| In Wasser dispergierbare Wirkstoffkonzentrate: | Spritzpulver (wettable powder), Pasten, Emulsionen, Emulsionskonzentrate; |
| Flüssige Aufarbeitungsformen: | Lösungen und Dispersionen. |

Die Wirkstoffkonzentrationen betragen in den erfindungsgemässen Mitteln 1 bis 80 Gewichtsprozent und können gegebenenfalls bei der

Anwendung auch in geringen Konzentrationen wie etwa 0,01 bis 1% vorliegen.

Den beschriebenen erfindungsgemässen Mitteln lassen sich andere für die Agrarchemie vorteilhafte Wirkstoffe oder Mittel beimischen, So können die neuen Mittel ausser den genannten Verbindungen der Formel I z.B. Insektizide, Fungizide, Bakterizide, Fungistatika, Bakteriostatika, Nematozide oder Herbizide zur Verbreiterung des Wirksungsspektrums enthalten.

Die nachfolgenden Beispiele beschreiben im Detail die Herstellung der Wirkstoffe wie auch erfindungsgemässer Mittel. In diesen Beispielen sind die Temperaturen in Centigraden angegeben, Teile und Prozentangaben beziehen sich auf das Gewicht. Druckangaben werden in millibar (mbar) gegeben.

<u>Beispiel 1:</u> Herstellung von N-(Pyrazol-1"-yl-1'-äthyl)-N-chloracetyl-2-chlor-6-methyl-anilin

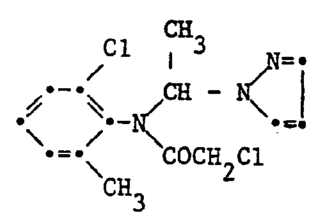

a.) In einem Sulfierkolben werden 121 ml 2-Chlor-6-methyl-anilin, 750 ml Benzol, 1 ml konz. Schwefelsäure und 160 g Molekularsieb 3Å vorgelegt. Unter Rühren und leichtem Kühlen tropft man bei 20-25°C langsam 85 ml wasserfreien Acetaldehyd zu. Nach 24 Stunden Rühren bei Raumtemperatur wird das Reaktionsgemisch filtriert und das Filtrat am Rotationsverdampfer eingeengt. Der Rückstand wird anschliessend am Wasserstrahlvakuum fraktioniert destilliert. Man erhält 60 g Schiff'sche Base der Formel

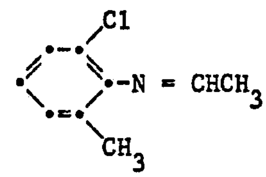

welche bei 98-99°C / 16 mbar siedet.

b.) Zu einer Lösung von 30 g dieser Schiff'schen Base in 150 ml Toluol lässt man bei -30°C unter Rühren 14 ml Chloracetylchlorid langsam zutropfen. Nach 30 Minuten tropft man bei gleicher Temperatur 27 g 1-Trimethylsilyl-pyrazol zu. Man lässt das Reaktionsgemisch über Nacht bei Raumtemperatur ausrühren und engt es dann am Vakuum ein. Der ölige Rückstand wird in Aether aufgenommen und auf Neutralkörper aufgearbeitet. Nach dem Verdampfen des Lösungsmittels im Vakuum wird das zurückbleibende Oel aus Diisopropyläther unter Zusatz von Tierkohle kristallisiert. Man erhält 20 g N-(Pyrazol-1"-yl-1'-äthyl)-N-chloracetyl-2-chlor-6-methyl-anilin vom Fp. 83-85°C.

In analoger Weise werden die folgenden Verbindungen hergestellt:

N-(Pyrazolyl-1-äth-1'-yl)-N-chloracetyl-2,6-dimethoxyanilin,Fp.116-117°
N-(Pyrazolyl-1-äth-1'-yl)-N-chloracetyl-2-methoxy-6-methylanilin,Fp.80-83°
N-(Pyrazolyl-1-äth-1'-yl)-N-chloracetyl-3-chlor-2,6-dimethylanilin
N-(Pyrazolyl-1-äth-1'-yl)-N-chloracetyl-2,6-dichloranilin,
Smp. 91-93°C
N-(Pyrazolyl-1-äth-1'-yl)-N-chloracetyl-2-chlor-6-methoxyanilin,
Smp. 101-104°C.

Beispiel 2: Herstellung von gebrauchsfertigen Anwendungsformen:

Granulat: Zur Herstellung eines 5%igen Granulates werden die folgenden Stoffe verwendet:

5 Teile N-(Pyrazolyl-1-äth-1'-yl)-N-chloracetyl-2-chlor-6-methylanilin,

0,25 Teile epoxydiertes Pflanzenöl,

0,25 Teile Cetylpolyglykoläther,
3,50 Teile Polyäthylenglykol,
91 Teile Kaolin (Korngrösse: 0,3-0,8 mm).

Die Aktivsubstanz wird mit dem Pflanzenöl vermischt und in 6 Teilen Aceton gelöst, hierauf wird Polyäthylenglykol und Cetylpolyglykoläther zugesetzt. Die so erhaltene Lösung wird auf Kaolin aufgesprüht und anschliessend im Vakuum verdampft.

Spritzpulver Zur Herstellung eines a) 70%igen und b) 10%igen Spritzpulvers werden folgende Bestandteile verwendet:

a) 70 Teile N-(Pyrazolyl-1-äth-1'-yl)-N-chloracetyl-2-chlor-6-methylanilin ,
5 Teile Natriumdibutylnaphthylsulfonat,
3 Teile Naphthalinsulfonsäuren-Phenolsulfonsäuren-Formaldehyd-Kondensat 3:2:1,
10 Teile Kaolin,
12 Teile Champagne-Kreide;

b) 10 Teile des obigen Wirkstoffes,
3 Teile Gemisch der Natriumsalze von gesättigten Fettalkoholsulfaten,
5 Teile Naphthalinsulfonsäuren-Formaldehyd-Kondensat,
82 Teile Kaolin.

Der Wirkstoff wird auf die entsprechenden Trägerstoffe (Kaolin und Kreide) aufgezogen und anschliessend vermischt und vermahlen. Man erhält Spritzpulver von vorzüglicher Benetzbarkeit und Schwebefähigkeit. Aus solchen Spritzpulvern können durch Verdünnen mit Wasser Suspensionen von 0,1-8% Wirkstoff erhalten werden, die sich z.B. zum Besprühen von Pflanzenkulturen, Rasen oder Pflanzenwuchs an Böschungen eignen.

Paste Zur Herstellung einer 45%igen Paste werden folgende Stoffe verwendet:

45 Teile N-(Pyrazolyl-1-äth-1'-yl)-N-chloracetyl-2-chlor-6-methylanilin,
5 Teile Natriumaluminiumsilikat,
14 Teile Cetylpolyglykoläther mit 8 Mol Aethylenoxid,
1 Teil Oleylpolyglykoläther mit 5 Mol Aethylenoxid,
2 Teile Spindelöl,
10 Teile Polyäthylenglykol,
23 Teile Wasser.

Der Wirkstoff wird mit den Zuschlagstoffen in dazu geeigneten Geräten innig vermischt und vermahlen. Man erhält eine Paste, aus der sich durch Verdünnen mit Wasser Suspensionen jeder gewünschten Konzentration herstellen lassen.

Emulsionskonzentrat Zur Herstellung eines 25%igen Emulsionskonzentrates werden

25 Teile N-(Pyrazolyl-1'-äthyl)-N-chloracetyl-2,6-dimethoxyanilin,
5 Teile einer Mischung von Nonylphenolpolyoxyäthylen und Calciumdodecylbenzolsulfonat,
35 Teile 3,3,5-Trimethyl-2-cyclohexan-1-on,
35 Teile Dimethylformamid

miteinander vermischt. Dieses Konzentrat kann mit Wasser zu Emulsionen auf geeignete Konzentrationen von z.B. 0,1 bis 10% verdünnt werden.

Beispiel 3: Nachweis der herbiziden und den Pflanzenwuchs regulierenden Wirkung.

Wuchshemmung bei Gräsern: In Kunststoffschalen mit Erde-Torf-Sand-Gemisch (6:3:1) wurden Samen der Gräser Lolium perenne, Poa pratenis, Festuca ovina und Dactylis glomerata ausgesät und normal bewässert.

Die aufgelaufenen Gräser wurden wöchentlich bis auf 4 cm zurückgeschnitten und 40 Tage nach der Aussaat und 1 Tag nach dem letzten Schnitt mit wässerigen Spritzbrühen des Wirkstoffes bespritzt. Die Wirkstoffmenge betrug umgerechnet 5 kg Aktivsubstanz pro Hektar. 10 und 21 Tage nach Applikation wurde das Wachstum der Gräser nach folgender Notenskala ausgewertet:

Note 1 = starke Hemmung (kein Weiterwchsen ab Applikationszeitpunkt, 0%)

Note 9 = keine Hemmung (100% Wachstum, wie unbehandelte Kontrolle).

Die geprüften N-(Pyrazolyl-1-äth-1'-yl)-N-chloracetyl-aniline bewirkten eine starke Wuchshemmung in diesem Versuch.

Wuchshemmung bei Getreide: In Kunststoffbechern wurde Sommerweizen (Triticum aestivum), Sommergerste (Hordeum vulgare) und Reis (Oryza sativa) in sterilisierter Erde angesät und im Gewächshaus gezogen. Weizen und Gerste wurden 5 Tage nach Aussaat, Reis 12 Tage nach Aussaat mit Spritzbrühen des Wirkstoffes behandelt. Die Blattapplikation entsprach 6 und 2 kg Wirkstoff pro Hektar (Bodenapplikation 500 und 100 ml Brühe pro Topf). Die Reisbecher wurden nach Applikation bis 3 cm über Erde mit Wasser beschichtet.

Die Auswertung erfolgte 7 und 21 Tage nach Applikation. N-(Pyrazolyl-1'-äthyl)-N-chloracetyl-2,6-dimethoxyanilin bewirkte eine deutliche Verkürzung und gleichzeitige Verstärkung des Halms.

Pre-emergente Herbizid-Wirkung (Keimhemmung

Im Gewächshaus wird unmittelbar nach der Einsaat der Versuchspflanzen in Töpfe die Erdoberfläche mit einer wässerigen Dispersion des Wirkstoffes, erhalten aus einem 25%igen Emulsionskonzentrat, behandelt. Es werden verschiedene Konzentrationsreihen angewendet, und die applizierte Wirkstoffmenge wird in kg Wirkstoffsubstanz pro Hektar

ausgerechnet. Töpfe werden im Gewächshaus bei 22-25°C und 50-70% rel. Luftfeuchtigkeit gehalten und der Versuch nach 3 Wochen ausgewertet.

Post-emergente Herbizid-Wirkung (Kontaktherbizid)

Eine grössere Anzahl Unkräuter und KUlturpflanzen, sowohl monocotyle wie dicotyle, wurden im Gewächshaus in Töpfen gezogen und nach dem Auflaufen (im 4- bis 6-Blattstadium) mit einer wässerigen Wirkstoffdispersion in verschiedenen Dosierungen, ausgedrückt in kg Wirksubstanz pro Hektare, auf die Pflanzen gespritzt und diese bei 24-26°C und 45-60% rel. Luftfeuchtigkeit gehalten. 15 Tage nach der Behandlung wird der Versuch ausgewertet.

Sowohl im Vor- wie im Nachauflaufversuch zeigte N-(Pyrazolyl-1'-äthyl)-N-chloracetyl-2-chlor-6-methoxyanilin gute Herbizidwirkung.

Patentansprüche:

1. N-(Azolyl-1-äth-1'-yl)-N-halogenacetanilide der Formel I

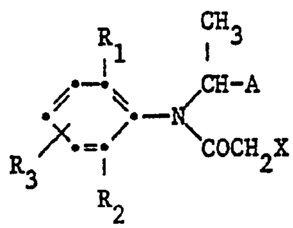

(I)

worin A einen über ein Stickstoffatom gebundenen Pyrazol-, Imidazol-, 1,2,4-Triazol- oder 1,3,4-Triazolring bedeutet, der durch $C_1$-$C_4$-Alkyl, Cyan oder Halogen substituiert sein kann, X ein Chlor- oder Bromatom und mindestens einer der Substituenten $R_1$, $R_2$, $R_3$, $C_1$-$C_4$-Alkoxy, Halogen oder Halogenmethyl, die anderen auch noch Wasserstoff oder $C_1$-$C_4$ Alkyl bedeuten, sowie die Säureadditionssalze dieser N-(Azolyl-1-äth-1'-yl)-N-halogenacetanilide.

2. N-(Pyrazolyl-1-äth-1'-yl)-N-chloracetanilide gemäss Anspruch 1 von der Formel Ia

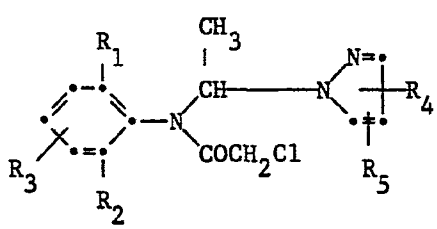

(Ia)

worin $R_1$, $R_2$ und $R_3$ die im Anspruch 1 unter Formel I gegebene Bedeutung haben und $R_4$ und $R_5$ unabhängig voneinander je $C_1$-$C_4$ Alkyl, Cyan oder Halogen bedeuten.

3. N-(Pyrazolyl-1"-äth-1'-yl)-N-chloracetyl-2,6-dimethoxyanilin.

4. N-(Pyrazolyl-1"-äth-1'-yl)-N-chloracetyl-2-methoxy-6-methylanilin.

5. N-(Pyrazolyl-1"-yl-äth-1'-yl)-N-chloracetyl-2-chlor-6-methylanilin.

6. N-(Pyrazolyl-1"-äth-1'-yl)-N-chloracetyl-3-chlor-2,6-dimethylanilin.

7. N-(Pyrazolyl-1"-äth-1'-yl)-N-chloracetyl-2-chlor-6-methoxyanilin.

8. N-(Pyrazolyl-1"-äth-1'-yl)-N-chloracetyl-2,6-dichloranilin.

9. Verfahren zur Herstellung der N-(Azolyl-1'-äth-1'-yl)-N-halogenacetanilide der Formel I, Anspruch 1, dadurch gekennzeichnet, dass man die Schiff'sche Base eines Anilins von der Formel II

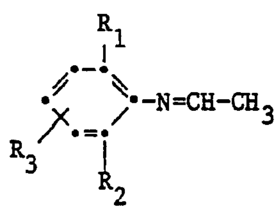

(II),

worin $R_1$, $R_2$ und $R_3$ die unter Formel I, Anspruch 1 gegebene Bedeutung haben, in einem inerten, aromatischen Lösungsmittel zuerst mit der äquimolaren Menge eines Halogenacetylhalogenides der Formel III

$$HalCOCH_2-X \qquad (III),$$

worin Hal und X unabhängig voneinander Chlor- oder Brom bedeuten, und anschliessend mit einem N-Trimethylsilylazol der Formel IV

$$(CH_3)_3\ Si-A \qquad (IV)$$

versetzt, worin A die unter Formel I, Anspruch 1 gegebene Bedeutung hat,und die erhaltene Verbindung gegebenenfalls in ein Säureadditionssalz umwandelt.

10. Herbizides und das Pflanzenwachstum regulierendes Mittel, dadurch gekennzeichnet, dass es als Wirkstoff mindestens ein N-(Azolyl-1-äth-1'-yl)-N-halogenacetanilid der Formel I, Anspruch 1 enthält.

11. Die Verwendung der N-(Azolyl-1-äth-1'-yl)-N-halogenacetanilide der Formel I, Anspruch 1 oder sie enthaltender Mittel als Herbizide.

12. Die Verwendung der N-(Azolyl-1-äth-1'-yl)-N-halogenacetanilide der Formel I, Anspruch 1 oder sie enthaltender Mittel zur Hemmung des Pflanzenwachstums.

Europäisches Patentamt

# EUROPÄISCHER RECHERCHENBERICHT

Nummer der Anmeldung

EP 80 81 0342.8

| EINSCHLÄGIGE DOKUMENTE | | | KLASSIFIKATION DER ANMELDUNG (Int. Cl.3) |
|---|---|---|---|
| Kategorie | Kennzeichnung des Dokuments mit Angabe, soweit erforderlich, der maßgeblichen Teile | betrifft Anspruch | |
| P | EP - A1 - 0 008 091 (BAYER)<br>* Ansprüche 1, 3 bis 5 *<br>-- | 1,2,<br>10-12 | C 07 D 231/12<br>A 01 N 43/56 |
| P | EP - A1 - 0 008 057 (BAYER)<br>* Seiten 24 bis 25; Tabelle 1 *<br>-- | 1,2 | |
| D | DE - A1 - 2 704 281 (BAYER)<br>* Ansprüche 3 bis 5 *<br>-- | 10-12 | |
| D | DE - A1 - 2 742 583 (BAYER)<br>* Ansprüche 3 bis 5 *<br>-- | 10-12 | RECHERCHIERTE SACHGEBIETE (Int. Cl.3) |
| P,A | DE - A1 - 2 854 598 (BASF)<br>---- | | A 01 N 43/56<br>C 07 D 231/12<br>C 07 D 233/56<br>C 07 D 233/61<br>C 07 D 249/08 |

KATEGORIE DER GENANNTEN DOKUMENTE

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

X Der vorliegende Recherchenbericht wurde für alle Patentansprüche erstellt.

| Recherchenort | Abschlußdatum der Recherche | Prüfer |
|---|---|---|
| Berlin | 18-02-1981 | FROELICH |